# EUROPEAN PATENT APPLICATION

(11) **EP 3 479 835 A1**
(43) Date of publication of application: **08.05.2019**
(21) Application number: 17820254.5
(22) Date of filing: 29.06.2017
(51) Int. Cl.: A61K 35/744, A23C 9/12, A23L 33/135, A61K 35/747, A61P 3/02

(54) **COMPOSITION FOR USE IN IMPROVEMENT OF NUTRITIONAL STATE**

(30) Priority: 30.06.2016 JP 2016130814
(71) Applicant: Asahi Group Holdings, Ltd., Tokyo 130-8602 (JP); Tokushima University, Tokushima 770-8501 (JP)
(72) Inventor: ROKUTAN Kazuhito, Tokushima-shi Tokushima 770-8501 (JP); NISHIDA Kensei, Tokushima-shi Tokushima 770-8501 (JP); KUWANO Yuki, Tokushima-shi Tokushima 770-8501 (JP); FUJIWARA Shigeru, Sagamihara-shi Kanagawa 252-0206 (JP); SUGAWARA Tomonori, Sagamihara-shi Kanagawa 252-0206 (JP); AOKI Yumeko, Sagamihara-shi Kanagawa 252-0206 (JP); SAWADA Daisuke, Sagamihara-shi Kanagawa 252-0206 (JP)
(74) Representative: D Young & Co LLP
(86) International application number: PCT/JP2017/023852
(87) International publication number: WO 2018/003900

(57) **Abstract**

This invention concerns a means and a method for improving a nutritional status. Specifically, this invention concerns a composition for improving the total serum protein level and/or the serum albumin level comprising, as an active ingredient, lactic acid bacteria and/or a treated product thereof, a composition for improving swelling, and a composition for increasing the muscle mass and/or the muscle strength.

## Description

### [Technical Field]

The present invention concerns a means and a method for improving a nutritional status. Specifically, the present invention concerns a composition for improving the total serum protein level and/or the serum albumin level comprising, as an active ingredient, lactic acid bacteria and/or a treated product thereof, a composition for improving swelling, and a composition for increasing the muscle mass and/or the muscle strength.

### [Background Art]

The total serum protein level or the serum albumin level (accounting for two-thirds of the total protein level) has been used as an indicator of a nutritional status, and such level has been known to decrease because of aging. In particular, albumin is a substance important for osmotic regulation in blood, which causes swelling. In addition, albumin plays a role in substance transport in a body, and a person with albumin deficiency cannot attain a sufficient benefit of exercise. In the case of elderly people, in particular, it becomes difficult to maintain the muscle strength.

At present, albumin deficiency is treated by administration of an albumin preparation or dietary instructions. Since an albumin preparation is expensive and administered via intravenous drips, a burden imposed on a patient is significant. While sufficient intake of proteins and amino acids is recommended by dietary instructions, proteins and amino acids are absorbed by the small intestine and hepatic or renal functions may have been deteriorated by aging or other reasons. So, the total serum protein or albumin level is considered to decrease even under dietary instructions. It is accordingly deduced that the total serum protein level and the serum albumin level can be increased by improving deteriorated hepatic or renal functions.

JP H09-110707 A describes that Lebenin®, which is a lactic acid bacteria preparation approved as a pharmaceutical mixture (the preparation comprising 10⁸ cfu *Bifidobacterium infantis*, 10⁸ cfu *Lactobacillus acidophilus*, and 10⁸ cfu *Enterococcus faecalis*), improves the intestinal microflora to thereby decrease indoxyl sulfate in blood, which is one of causes of chronic renal failure.

JP 2012-017282 A describes that *Lactobacillus gasseri* strain CP2305 has effects of activating the vagus nerve and effects of relieving stress.

JP 2014-055195 A describes that viable cells of *Lactobacillus gasseri* strain SBT2055 have effects of preventing and improving deteriorated renal functions.

WO 2013/031749 A describes that activity level and muscle mass of a mouse is increased through administration of dead bacterial cells of *Lactobacillus gasseri* strain OLL2809 to the mouse.

### [Summary of the Invention]

### [Objects to Be Attained by the Invention]

It is an object of the present invention to provide a means and a method that are effective for improving a nutritional status of, in particular, middle-aged to older people and subjects with a deteriorated nutritional status. It is another object of the present invention to provide a means and a method for suppressing swelling caused by a poor nutritional status and maintaining and improving the muscle strength.

### [Means for Attaining the Objects]

The present inventors have conducted concentrated studies in order to attain the above objects. As a result, they found that the nutritional status of middle-aged to older people or subjects with a deteriorated nutritional status could be improved by administering lactic acid bacteria (*Lactobacillus gasseri*, in particular) thereto. Specifically, they found that the total serum protein level and the albumin level could be improved, swelling could be suppressed, and/or the muscle strength and the muscle mass could be maintained or improved through administration of lactic acid bacteria. In addition, they discovered that dead bacterial cells of the lactic acid bacteria used herein could exert the effects of interest.

The present invention includes, but is not limited to, the following.
[1] A composition for improving the total serum protein level and/or the serum albumin level comprising, as an active ingredient, lactic acid bacteria and/or a treated product thereof.
[2] A composition for improving swelling comprising, as an active ingredient, lactic acid bacteria and/or a treated product thereof.
[2-1] The composition according to [2], wherein the swelling occurs in the triceps muscle of the calf.
[3] A composition for increasing the muscle mass and/or the muscle strength comprising, as an active ingredient, lactic acid bacteria and/or a treated product thereof.
[4] The composition according to any of [1] to [3], wherein the lactic acid bacteria belong to a genus selected from the group consisting of the genera *Lactobacillus*, *Leuconostoc*, *Lactococcus*, *Pediococcus*, *Enterococcus*, *Bifidobacterium*, *Streptococcus*, and *Weissella.*
[5] The composition according to any of [1] to [4], wherein the lactic acid bacteria are *Lactobacillus gasseri.*
[6] The composition according to any of [1] to [5], wherein the lactic acid bacteria are *Lactobacillus gasseri* strain CP2305 (Accession Number: FERM BP-11331) or a variant thereof, or *Lactobacillus gasseri* strain CP2305s (Accession Number: NITE BP-1405) or a variant thereof.
[6-1] The composition according to any of [1] to [6], wherein the lactic acid bacteria are dead bacterial cells.
[7] The composition according to any of [1] to [6], which is used for improving a nutritional status.
[8] The composition according to any of [1] to [7], which is administered to human subjects in their 40s or older.
[9] The composition according to any of [1] to [8], which is administered to human subjects with the total serum protein level of 6.5 g/dL or lower and/or the serum albumin level of 4 g/dL or lower.
[10] The composition according to any of [1] to [9], which is selected from the group consisting of food or drink products, feeds, nutritious supplements, and medicaments.
[11] The composition according to [10], wherein the food or drink products comprise fermented milk beverages, yogurt, powdered milk, baby foods, *miso* soup, retort foods, and tablets.
[12] A method of improving the total serum protein level and/or the serum albumin level in a subject comprising a step of administering lactic acid bacteria and/or a treated product thereof to the subject.
[12-1] The method according to [12], wherein the total serum protein level and/or the serum albumin level of the subject are increased, compared with a control group to which the lactic acid bacteria and/or the treated product thereof are not administered.
[13] A method of improving swelling in a subject comprising a step of administering lactic acid bacteria and/or a treated product thereof to the subject.
[13-1] The method according to [13], wherein the swelling occurs in the triceps muscle of the calf.
[13-2] The method according to [13], wherein the swelling is reduced, compared with a control group to which the lactic acid bacteria and/or the treated product thereof are not administered.
[14] A method of increasing the muscle mass and/or the muscle strength in a subject comprising a step of administering lactic acid bacteria and/or a treated product thereof to the subject.
[14-1] The method according to [14], wherein the muscle mass and/or the muscle strength of the subject are increased, compared with a control group to which the lactic acid bacteria and/or the treated product thereof are not administered.
[15] A method of improving a nutritional status in a subject comprising a step of administering lactic acid bacteria and/or a treated product thereof to the subject to improve the total serum protein level and/or the serum albumin level in the subject.
[16] A method of improving a nutritional status in a subject comprising a step of administering lactic acid bacteria and/or a treated product thereof to the subject to improve swelling of the subject.
[17] A method of improving a nutritional status in a subject comprising a step of administering lactic acid bacteria and/or a treated product thereof to the subject to increase the muscle mass and/or the muscle strength of the subject.

### [Effects of the Invention]

The present invention provides a composition for improving the total serum protein level and/or the serum albumin level, a composition for improving swelling, and a composition for increasing the muscle mass and/or the muscle strength. The composition of the present disclosure is particularly effective for improving a nutritional status of middle-aged to older people and subjects with a poor nutritional status. In addition, lactic acid bacteria as active ingredients are safe and cost-effective, and intake thereof in the form of food or drink products or nutritious supplements can be easy.

### [Brief Description of the Drawings]

Fig. 1 is a graph showing effects of lactic acid bacteria for improving the serum albumin level.
Fig. 2 is a graph showing effects of lactic acid bacteria for improving the total serum protein level.
Fig. 3 is a graph showing effects of lactic acid bacteria for improving swelling (i.e., the circumferential length of the triceps muscle of the calf).
Fig. 4 is a graph showing effects of lactic acid bacteria for improving the growth hormone level.
Fig. 5 is a graph showing effects of lactic acid bacteria for improving the IGF-1 level.
Fig. 6 is a graph showing effects of lactic acid bacteria for improving the circumferential length of the gastrocnemius muscle.
Fig. 7 is a graph showing effects of lactic acid bacteria for improving the grip strength.

### [Embodiments for Carrying out the Invention]

The present invention is based on findings that administration of lactic acid bacteria can improve the total serum protein level and/or the serum albumin level, improve swelling, or increase the muscle mass and/or the muscle strength, and it can improve a nutritional status. Specifically, the present invention is based on the finding that administration of lactic acid bacteria (i.e., *Lactobacillus gasseri* strain CP2305) to the subjects of middle-aged to older people (people in their 40s to 90s) would exert effects of improving the total serum protein level or the serum albumin level, compared with placebo samples, and lactic acid bacteria would exert effects of improving a nutritional status.

Accordingly, the present invention concerns a composition for improving the total serum protein level and/or the serum albumin level comprising, as an active ingredient, lactic acid bacteria and/or a treated product thereof, a composition for improving swelling, and a composition for increasing the muscle mass and/or the muscle strength. In addition, the present invention concerns a method of improving the total serum protein level and/or the serum albumin level in a subject, a method of improving swelling in a subject, and a method of increasing the muscle mass and/or the muscle strength in a subject comprising a step of administering lactic acid bacteria and/or a treated product thereof to the subject.

The lactic acid bacteria used in the present invention are capable of producing lactic acid from sugars by fermentation. Examples thereof include bacteria belonging to the genera *Lactobacillus*, *Leuconostoc*, *Lactococcus*, *Pediococcus*, *Enterococcus*, *Bifidobacterium*, *Streptococcus*, and *Weissella.* According to the present invention, bacterial cells of lactic acid bacteria known in the art can be used, as long as bacterial cells or a treated product of lactic acid bacteria exert the function or activity of interest specifically described below. In addition, bacterial strains that have been confirmed to be safe for animals may be preferable in terms of administration to or intake by animals.

Specific examples of lactic acid bacteria include bacteria belonging to the genus *Lactobacillus*, such as *Lactobacillus gasseri*, *Lactobacillus amylovorus*, *Lactobacillus casei*, *Lactobacillus paracasei*, *Lactobacillus zeae*, *Lactobacillus rhamnosus*, *Lactobacillus reuteri*, *Lactobacillus acidophilus*, *Lactobacillus crispatus*, *Lactobacillus gallinarum*, *Lactobacillus brevis*, *Lactobacillus fermentum*, *Lactobacillus plantarum*, *Lactobacillus delbrueckii subsp. bulgaricus*, and *Lactobacillus johnsonii.*

Other specific examples of lactic acid bacteria include bacteria belonging to the genus *Bifidobacterium*, such as *Bifidobacterium breve*, *Bifidobacterium longum*, *Bifidobacterium pseudolongum*, *Bifidobacterium animalis*, *Bifidobacterium adolescentis*, *Bifidobacterium bifidum*, *Bifidobacterium lactis*, *Bifidobacterium catenulatum*, *Bifidobacterium pseudocatenulatum*, and *Bifidobacterium magnum.* Examples of bacteria belonging to the genus *Enterococcus* include *Enterococcus faecalis*, *Enterococcus hirae,* and *Enterococcus faecium.* An example of bacteria belonging to the genus *Streptococcus* is *Streptococcus thermophilus.* Examples of bacteria belonging to the genus *Leuconostoc* include *Leuconostoc mesenteroides* and *Leuconostoc lactis.* Examples of bacteria belonging to the genus *Lactococcus* include *Lactococcus lactis*, *Lactococcus plantarum*, and *Lactococcus raffinolactis.* Examples of bacteria belonging to the genus *Pediococcus* include *Pediococcus pentosaceus* and *Pediococcus damnosus.* Examples of bacteria belonging to the genus *Weissella* include *Weissella cibaria*, *Weissella confusa*, *Weissella halotolerans*, *Weissella hellenica*, *Weissella kandleri*, *Weissella kimchii*, *Weissella koreensis*, *Weissella minor*, *Weissella paramesenteroides*, *Weissella soli*, *Weissella thailandensis*, and *Weissella viridescens.*

According to an embodiment of the present invention, the function or activity of interest may be activity of improving the total serum protein level and/or the serum albumin level in a subject. In this case, the term "improving" refers to increasing the total serum protein level and/or the serum albumin level, approximating the total serum protein level and/or the serum albumin level to normal levels (e.g., 6.5 to 8.3 g/dL and 3.5 to 5.2 g/dL, respectively), and preventing the total serum protein level and/or the serum albumin level from decreasing. The total serum protein level and the serum albumin level can be measured by any method known in the art.

According to another embodiment of the present invention, the function or activity of interest may be activity of improving swelling. Swelling (edema) is a condition caused by an abnormal increase of fluid in the body, which develops throughout the body including the face, the limbs, and visceral organs. In this case, the term "improving" refers to suppressing swelling and decreasing the occurrence of swelling. Swelling can be measured by any method known in the art. For example, swelling can be measured based on a change in the circumferential length of the triceps muscle of the calf, a change in the circumferential length of the ankle, and a change in a duration during which a dimple remains in the area below the knee after being pressed.

According to another embodiment of the present invention, the function or activity of interest may be activity of increasing the muscle mass and/or the muscle strength. In this case, the term "increasing" refers to not only increasing the muscle mass and/or the muscle strength but also preventing the muscle mass and/or the muscle strength from decreasing. An increased muscle mass can be evaluated by, for example, measuring a level of muscle-associated factors (e.g., the growth hormone and the insulin-like growth factor 1 (IGF-1)), and increased muscle strength can be evaluated by, for example, measuring the circumferential length of the gastrocnemius muscle or the grip strength.

According to a particular embodiment of the present invention, the function or activity of interest may be activity of improving a nutritional status. By improving a nutritional status, the metabolism that maintains energy and a structure necessary for activity of maintenance of physical functions can be maintained in a normal state. A nutritional status can be improved by increasing the blood hemoglobin level, such as the total serum albumin level and/or the total serum protein level. In a deteriorated nutritional status, the metabolism that maintains energy and a structure necessary for activity of maintenance of physical functions is not maintained in a normal state. When the total serum protein level is 6.5 g/dL or lower and/or the serum albumin level is 3.5 g/dL or 4 g/dL or lower, for example, the nutritional status of the subject may bes evaluated to be deteriorated or low even in a normal state.

According to the present invention, any lactic acid bacteria can be used, as long as bacterial cells or a treated product of lactic acid bacteria are evaluated to have the function or activity of interest by the method described above. Examples of preferable lactic acid bacteria having such function or activity include *Lactobacillus gasseri* strain CP2305 and *Lactobacillus gasseri* strain CP2305s. *Lactobacillus gasseri* strain CP2305 is deposited internationally by the applicant under Accession Number FERM BP-11331 as of September 11, 2007 with the International Patent Organism Depositary, the National Institute of Advanced Industrial Science and Technology (AIST) (Tsukuba Central 6, 1-1-1 Higashi, Tsukuba, Ibaraki, Japan), which is the international depositary authority under the terms of the Budapest Treaty for deposition of patent microorganisms. At present, this strain is transferred to and stored at the Patent Microorganisms Depositary, the National Institute of Technology and Evaluation (120, 2-5-8, Kazusakamatari, Kisarazu-shi, Chiba, Japan), which is the international depositary authority. Also, *Lactobacillus gasseri* strain CP2305s is deposited internationally by the applicant under Accession Number NITE BP-1405 as of August 14, 2012 with the Patent Microorganisms Depositary, the National Institute of Technology and Evaluation (122, 2-5-8, Kazusakamatari, Kisarazu-shi, Chiba, Japan), which is the international depositary authority under the terms of the Budapest Treaty for deposition of patent microorganisms.

According to the present invention, variants of the specific strains mentioned above can also be used, as long as they have the function or activity of interest. For example, *Lactobacillus gasseri* strain CP2305s is a variant obtained from *Lactobacillus gasseri* strain CP2305, variants of the strain CP2305 and the strain CP2305s are highly likely to have the function or activity of interest, and such variants can also be used in the present invention.

The term "variant" used herein refers to any strain obtained from a parent strain. Specifically, this term refers to a strain obtained by a method of artificially increasing the frequency of mutation from a parent strain by means of spontaneous mutation or mutagenesis with chemical or physical mutagens or a strain obtained by a specific mutagenesis technique (e.g., gene recombination). Microbial individuals resulting from such techniques may be repeatedly subjected to selection and separation, and variants with the function or activity of interest can be obtained by breeding of useful microbial individuals.

For example, variants originating from *Lactobacillus gasseri* strain CP2305 or CP2305s can be easily distinguished from other lactic acid bacterial strains based on the molecular weight distribution of amplified genomic DNA fragments of lactic acid bacteria determined by the polymerase chain reaction (PCR). In short, DNA samples of lactic acid bacteria of interest are prepared, gene amplification is carried out by PCR using primers having unique sequence (e.g., 16S rDNA-derived nucleotide sequence), and electrophoresis patterns of the obtained fragments are analyzed. Thus, whether or not the lactic acid bacterial strain of interest is a variant originating from *Lactobacillus gasseri* strain CP2305 or CP2305s can be determined. It should be noted that the technique of confirming whether or not the bacterial strain of interest is a variant is not limited to the above, and whether or not the bacterial strain of interest is a variant can be confirmed by a technique known in the art based on, for example, mycological properties.

Lactic acid bacteria can be prepared via culture under adequate conditions using any of media conventionally used for culture of the lactic acid bacteria. In this case, a natural or synthetic medium can be used, as long as it contains a carbon source, a nitrogen source, mineral salts, and the like, and lactic acid bacteria can be cultured efficiently therein. A person skilled in the art can adequately select a known medium suitable for a bacterial strain to be used. Examples of the carbon source that can be used include lactose, glucose, sucrose, fructose, galactose, and blackstrap molasses. Examples of the nitrogen source that can be used include organic nitrogen-containing substances such as casein hydrolysate, whey protein hydrolysate, and soy protein hydrolysate. In addition, examples of the mineral salts that can be used include phosphate, sodium, potassium, and magnesium. Examples of media suitable for culture of lactic acid bacteria include MRS liquid medium, GAM medium, BL medium, Briggs Liver Broth, animal milk, skim milk, and milk-derived whey. Preferably, sterilized MRS medium can be used.

Culture of lactic acid bacteria can be performed at 20°C to 50°C, preferably at 25°C to 42°C, and more preferably at approximately 37°C under anaerobic conditions. Temperature conditions can be adjusted using a thermostatic bath, a mantle heater, a jacket, or the like. The term "anaerobic conditions" used herein refers to a low-oxygen environment in which lactic acid bacteria can proliferate. For example, anaerobic conditions can be provided by using an anaerobic chamber, anaerobic box, or airtight container or bag containing a deoxidizer, or by simply sealing a culture container. Examples of culture formats include static culture, shake culture, and tank culture. The period of culture can be 3 hours to 96 hours. It may be preferable to maintain the pH of a medium at 4.0 to 8.0 in the beginning of culture.

Specific examples of lactic acid bacteria preparation are briefly described below. When *Lactobacillus gasseri* strain CP2305 or CP2305s is used, for example, lactic acid bacteria are inoculated into a medium for lactic acid bacteria culture (e.g., an MRS liquid medium) and preferably a food-grade medium for lactic acid bacteria culture, and culture may be carried out overnight (for approximately 18 hours) at approximately 37°C.

After culture, the obtained culture product of lactic acid bacteria can be used in that state, or it may be further subjected to, for example, crude purification via centrifugation and/or solid-liquid separation via filtration and sterilization, according to need. In addition, lactic acid bacteria used in the present invention may be in the form of viable bacterial cells or dead bacterial cells and/or in the form of wet bacterial cells or dried bacterial cells. Use of dead lactic acid bacteria may be preferable.

In addition, a treated product of lactic acid bacteria obtained by treating bacterial cells of lactic acid bacteria may be used, as long as it has the function or activity of interest. Alternatively, a treated product of lactic acid bacteria may be further subjected to treatment. Examples of such treatment are described below.

Bacterial cells and/or a treated product of lactic acid bacteria can be prepared in the form of a suspension or diluted solution by suspension or dilution in an adequate solvent. Examples of a solvent that can be used include water, physiological saline, and phosphate buffer saline (PBS).

A fermentation product can be prepared by fermenting raw milk, skim milk, or soymilk using bacterial cells and/or a treated product of lactic acid bacteria. For example, lactic acid bacteria or lactic acid bacteria subjected to optional treatment may be inoculated into raw milk, skim milk, or soymilk, followed by fermentation under conditions (substantially equivalent to the above conditions for culture of lactic acid bacteria) known in the art. The thus obtained fermentation product can be used in that state, or it may be subjected to optional treatment such as filtration, sterilization, dilution, or concentration.

A sterilized product of lactic acid bacteria can be prepared by sterilization treatment of bacterial cells and/or a treated product of lactic acid bacteria. In order to subject bacterial cells and/or a treated product of lactic acid bacteria to sterilization treatment, for example, a known technique of sterilization treatment such as filtration sterilization, radiation sterilization, heat sterilization, or high pressure sterilization can be used.

In addition, bacterial cells and/or a treated product of lactic acid bacteria can be subjected to heat treatment so as to prepare a heated product of lactic acid bacteria. In order to prepare such heated product, bacterial cells and/or a treated product of lactic acid bacteria are(is) subjected to high-temperature treatment (for example, at 80°C to 150°C) for a certain period of time, such as approximately 10 minutes to 1 hour (e.g., approximately 10 to 20 minutes).

Bacterial cells and/or a treated product of lactic acid bacteria can be subjected to disruption, fracturing, or grinding to prepare a disrupted product or a cell-free extract. For example, physical disruption (e.g., agitation or filter filtration), enzymatic lysis treatment, chemical treatment, or autolysis treatment can be performed.

Bacterial cells and/or a treated product of lactic acid bacteria can be subjected to extraction with the use of an adequate aqueous or organic solvent to obtain an extract. A method of extraction is not particularly limited, as long as such method involves the use of an aqueous or organic solvent as an extraction solvent. For example, a known method, such as a method comprising immersing the lactic acid bacteria or lactic acid bacteria subjected to optional treatment in an aqueous or organic solvent (e.g., water, methanol, or ethanol), or agitating or refluxing the lactic acid bacteria or lactic acid bacteria subjected to optional treatment in the solvent can be adopted.

Also, bacterial cells and/or a treated product of lactic acid bacteria can be subjected to drying to process into the form of a powdery product (powder) or granular product. Specific examples of drying methods include, but are not particularly limited to, spray drying, drum drying, vacuum drying, and freeze-drying, which can be used alone or in combination. Upon drying, a conventional excipient may be added, according to need.

In addition, bacterial cells and/or a treated product of lactic acid bacteria can be subjected to known separation/purification techniques to purify an ingredient or fraction having the function or activity of interest. Examples of such separation/purification techniques include: a method involving salt precipitation or organic solvent precipitation in accordance with degrees of solubility; a method involving dialysis, ultrafiltration, or gel filtration in accordance with molecular weight differences; a method involving ion-exchange chromatography in accordance with charge differences; a method involving affinity chromatography in accordance with degrees of specific binding; and a method involving hydrophobic chromatography or reversed-phase chromatography in accordance with degrees of hydrophobicity, which can be used alone or in combinations of two or more thereof.

The treatments described above may be carried out alone or in combinations of two or more thereof. According to the present invention, such treated products can be used in the composition of the present disclosure.

Through continuous intake of the bacterial cells and/or a treated product of lactic acid bacteria obtained above alone or in combination with other ingredients in the form of the composition of the present disclosure, food or drink products, feeds, nutritious supplements, or pharmaceutical compositions, effects of improving the total serum protein level and/or the serum albumin level, effects of improving swelling, or effects of increasing the muscle mass and/or the muscle strength, and effects of improving a nutritional status attained by the effects mentioned above can be contemplated.

The composition of the present disclosure comprises, as an active ingredient, the bacterial cells and/or a treated product of lactic acid bacteria described above. Such composition may comprise a single type of bacterial cells and/or a treated product of lactic acid bacteria, a plurality of different types of bacterial cells and/or a treated product of lactic acid bacteria, or treated products of a plurality of lactic acid bacteria subjected to different treatments in combination.

In addition to the bacterial cells and/or a treated product of lactic acid bacteria as active ingredients, the composition of the present disclosure may be supplemented with the additives described below, other known drugs, or nutritious supplements alone or in combinations of two or more, as long as the function or activity of interest is not inhibited.

While the dosage form of the composition of the present disclosure is not particularly limited, examples of dosage forms include: oral formulations such as tablets, capsules, granules, powders, dust formulations, syrups, dry syrups, solutions, suspensions, and inhalers; enteral formulations such as suppositories; infusions; and parenteral injections. Among them, an oral formulation may be preferable. In addition, a liquid formulation, such as a solution or suspension, may be dissolved or suspended in water or a different adequate medium immediately before use. In the case of tablets or granules, surfaces thereof may be coated by a well-known method. Further, the composition of the present disclosure may be prepared in the form of a controlled-release formulation, such as a sustained-release formulation, a delayed-release formulation, or an immediate-release formulation, by a technique known in the art.

The composition in the dosage form described above can be prepared in accordance with a conventional method by formulating conventional additives, such as excipients, disintegrants, binders, wetting agents, stabilizers, buffering agents, lubricants, preservatives, surfactants, sweeteners, flavoring agents, aromatics, acidulants, and coloring agents, into the ingredients described above in accordance with the dosage form. When the composition of the present disclosure is prepared in the form of a pharmaceutical composition, for example, a pharmaceutically acceptable carrier or additive can be incorporated into the composition. Examples of such pharmaceutically acceptable carriers and additives include water, pharmaceutically acceptable organic solvents, collagen, polyvinyl alcohol, polyvinyl pyrrolidone, carboxyvinyl polymers, sodium alginate, water-soluble dextran, water-soluble dextrin, sodium carboxymethyl starch, pectin, xanthan gum, gum Arabic, casein, gelatin, agar, glycerin, propylene glycol, polyethylene glycol, vaseline, paraffin, stearyl alcohol, stearic acid, human serum albumin, mannitol, sorbitol, lactose, surfactants acceptable as pharmaceutical additives, and artificial cell constructs such as liposomes.

When the composition of the present disclosure comprises the additives described above or other drugs, the content of the bacterial cells and/or a treated product of lactic acid bacteria as active ingredients varies depending on the dosage form. The content of lactic acid bacteria may be generally 0.0001% to 99% by mass, preferably 0.001% to 80% by mass, and more preferably 0.001% to 75% by mass. It may be preferable that the composition be prepared into a dosage form that allows management of the daily dose, so as to achieve intake of the active ingredients in preferable amounts. In addition, the number of lactic acid bacteria or a treated product thereof to be incorporated into the composition of the present disclosure may be approximately 10⁷ cells/g to approximately 10¹² cells/g (when counted as the number of bacterial cells of lactic acid bacteria before treatment).

Examples of other drugs that can be added to or incorporated into the composition of the present disclosure include, but are not limited to, taurine, coenzyme Q, polyphenols, catechins, animal proteins, milk proteins, and plant proteins.

The composition of the present disclosure may further contain a variety of additives used for production of medicaments, food or drink products, and feeds and other various substances. Examples of such substances and additives include a variety of fats and oils (e.g., plant oils, such as soybean oil, corn oil, safflower oil, and olive oil, and animal fats and oils, such as beef fat and sardine oil), crude drugs (e.g., royal jelly and ginseng), amino acids (e.g., glutamine, cysteine, leucine, and arginine), polyalcohols (e.g., ethylene glycol, polyethylene glycol, propylene glycol, glycerin, and sugar alcohols such as sorbitol, erythritol, xylitol, maltitol, and mannitol), natural polymers (e.g., gum Arabic, agar, water-soluble corn fibers, gelatin, xanthan gum, casein, gluten or gluten hydrolysate, lecithin, starch, and dextrin), vitamins (e.g., vitamin C and B-complex vitamins), minerals (e.g., calcium, magnesium, zinc, and iron), dietary fibers (e.g., mannan, pectin, and hemicellulose), surfactants (e.g., glycerin fatty acid esters and sorbitan fatty acid esters), purified water, excipients (e.g., glucose, cornstarch, lactose, and dextrin), stabilizers, pH-adjusting agents, antioxidants, sweeteners, taste components, acidulants, coloring agents, and flavors.

The amount of such additive can be adequately determined depending on the type of additive and the desirable amount to be intaken. The content of bacterial cells and/or a treated product of lactic acid bacteria as active ingredients may vary depending on the dosage form. The content may be generally 0.0001% to 99% by mass, preferably 0.001% to 80% by mass, and more preferably 0.001% to 75% by mass, as the amount of the lactic acid bacteria before treatment.

Subjects of administration or intake of the composition of the present disclosure may be vertebrate animals. Specific examples thereof include mammals such as humans, primates (e.g., monkeys and chimpanzees), livestock animals (e.g., cattle, horses, pigs, sheep, and chickens), pet animals (e.g., dogs and cats), and experimental animals (e.g., mice and rats). Further, subjects can be reptiles and birds. Particularly preferable subjects may be those for whom the nutritional status is expected to improve, such as middle-aged to older people (e.g., people in their 40s or older) and humans with a deteriorated or poor nutritional status (e.g., humans with the total serum protein level of about 6.5 g/dL or lower and/or the serum albumin level of about 4 g/dL or lower).

The dose of administration or intake of the composition of the present disclosure may vary depending on the age and the body weight of a subject, the administration/intake route, the administration/intake frequency, the purpose of administration (e.g., improvement of a nutritional status, improvement of the total serum protein level and the albumin level, improvement of swelling, and increase of the muscle mass and the muscle strength), and other conditions. The dose of administration or intake can be changed extensively at the discretion of a person skilled in the art to achieve a desired effect. For oral administration or intake, for example, it may be preferable to administer bacterial cells and/or a treated product of lactic acid bacteria contained in the composition in an amount of generally approximately 10⁶ cells to 10¹² cells, and preferably approximately 10⁷ cells to 10¹¹ cells per kg of body weight, as the amount of the bacterial cells of lactic acid bacteria. The content of bacterial cells and/or a treated product of lactic acid bacteria is not particularly limited and can be adequately adjusted in accordance with the ease of production, the preferable daily dose, or other conditions. Since the composition of the present disclosure is highly safe, it is also possible to further increase the dose to be administered. A daily dose may be administered in a single instance or in several separate instances. In addition, the frequency of administration or intake is not particularly limited, and it can be adequately selected depending on various conditions, such as the route of administration or intake, age or body weight of a subject, and desired effects (e.g., improvement of a nutritional status, improvement of the total serum protein level and the albumin level, improvement of swelling, and increase of the muscle mass and the muscle strength).

The administration or intake route of the composition of the present disclosure is not particularly limited, and it can be, for example, oral administration or intake or parenteral administration (e.g., intrarectal, subcutaneous, intramuscular, or intravenous administration). Particularly preferably, the composition of the present disclosure may be orally administered or ingested.

The composition of the present disclosure improves the deteriorated hepatic or renal function to thereby improve the total serum protein level and/or the serum albumin level. In particular, the composition may be effective for the lowered total serum protein level and/or the serum albumin level caused by aging. Specifically, the total serum protein level and/or the serum albumin level may be increased in the subject, compared with a control to which the composition of the present disclosure (lactic acid bacteria and/or a treated product thereof) is not administered. Also, the composition of the present disclosure may improve swelling or increase the muscle mass and/or the muscle strength. Specifically, swelling may be reduced or the muscle mass and/or the muscle strength may be increased in the subject, compared with a control to which the composition of the present disclosure (lactic acid bacteria and/or a treated product thereof) is not administered. Thus, the composition of the present disclosure has effects of improving a nutritional status in a subject, in particular, a middle-aged or older individual or a subject with a deteriorated nutritional status.

The composition of the present disclosure may be used in combination with an other medicament or an other therapeutic or preventive method. The "other medicament" and the composition of the present disclosure may be formulated into a single formulation. Alternatively, they may be formulated into separate formulations to administer them simultaneously or at intervals.

As described above, the composition of the present disclosure can be used in the form of a pharmaceutical composition for improvement of the total serum protein level and the serum albumin level, improvement of swelling, increase of the muscle mass and the muscle strength, improvement of a nutritional status, and other purposes.

In addition, the composition of the present disclosure is highly safe and thus is easily used for long-term continuous intake. Therefore, the composition of the present disclosure can also be added to food or drink products, nutritious supplements, or feeds. The composition of the present disclosure has the function or activity described above, and it contains lactic acid bacteria that have been used for meals. Thus, such composition is highly safe. Further, even when it is added to a variety of food or drink products, it does not inhibit the flavor of a food or drink product itself. Thus, it can be added to various types of food or drink products and continuously ingested. As a consequence, the function or activity of interest can be expected.

The food or drink product of the present disclosure contains the composition of the present disclosure as described above. The food or drink product of the present disclosure also includes beverages. Examples of the food or drink product containing the composition of the present disclosure include functional food or drink products aimed at better health by functions or activity, including improvement of a nutritional status, improvement of the total serum protein level and the albumin level, improvement of swelling, and increase of the muscle mass and the muscle strength (e.g., nutritious supplements, food for specified health use, foods with nutrient function claims, and foods with functional claims), and all food or drink products into which the composition of the present disclosure can be incorporated.

Functional food or drink products are particularly preferable as food or drink products containing the composition of the present disclosure. The "functional food or drink product" of the present disclosure means a food or drink product having a predetermined function for organisms and encompasses, for example, all of so-called health food or drink products such as food or drink products with health claims including foods for specified health use (FOSHU) and food or drink products with nutrient function claims, foods with function claims, foods for special dietary uses, nutritional supplements, health supplements, supplements (e.g., those having a variety of dosage forms such as tablets, coated tablets, sugar-coated tablets, capsules, and liquid agents), and beauty food or drink products (e.g., diet food or drink products). The functional food or drink products of the present disclosure also encompass health food or drink products to which health claim based on Codex (Joint FAO/WHO Food Standards Programme) food standards are applied.

Specific examples of food or drink products include: health food or drink products and nutritional supplements in preparation forms such as liquid diets (e.g., tube enteral nutritional supplements), tablet candies, tablets, chewable tablets, tablets, dust formulations, powders, capsules, granules, and tonic drinks; tea beverages such as green tea, oolong tea, and black tea; beverage products such as soft drinks, jelly beverages, sport beverages, milk beverages, carbonated beverages, vegetable beverages, juice beverages, fermented vegetable beverages, fermented fruit juice beverages, fermented milk beverages (e.g., yogurt), lactic acid bacteria beverages, milk beverages (e.g., coffee milk and fruit milk), beverages containing drink powders (e.g., milk formula), cocoa beverages, milk, and purified water; spreads such as butter, jam, dried seasoning products, and margarine; mayonnaise; shortening; custard; dressings; breads; boiled rice; noodles; pasta; Japanese *miso* soup; Japanese *tofu*; yogurt; baby foods; soups or sauces; and sweets (e.g., biscuits and cookies, chocolates, candies, cakes, ice creams, chewing gums, and tablets).

The food or drink product of the present disclosure can be produced by conventional methods by adding other food materials used for production of the above-mentioned food or drink products, various nutrients, various vitamins, minerals, dietary fibers, and various additives (e.g., taste components, sweeteners, acidulants such as organic acids, stabilizers, and flavors), in addition to the above-mentioned active ingredients.

For the food or drink product of the present disclosure, a person skilled in the art can adequately determine the amount of the lactic acid bacteria and/or a treated product thereof as an active ingredient to be incorporated thereinto by taking the form of the food or drink product and the desirable taste or texture into consideration. In general, a person skilled in the art may appropriately determine the total amount of bacterial cells and/or a treated product of lactic acid bacteria to be added to the composition, so as to adjust the amount of lactic acid bacteria to 0.0001% to 99% by mass, preferably 0.001% to 80% by mass, and more preferably 0.001% to 75% by mass. The composition of the present disclosure is highly safe. As such, the amount of the composition incorporated into a food or drink product can be further increased. In order to achieve consumption of the desirable amount of the composition, it may be desirable to prepare the composition in a dosage form that allows management of the daily dose. As described above, the food or drink product of the present disclosure can be consumed in a manner that allows management of a desirable amount of the composition of the present disclosure. Thus, a method for improving a nutritional status, a method for improving the total serum protein level and the albumin level, a method for improving swelling, and a method for increasing the muscle mass and the muscle strength using such food or drink products can be provided.

The composition of the present disclosure may be incorporated into a food or drink product by any appropriate method available to a person skilled in the art. For example, the composition of the present disclosure can be prepared in the liquid, gel, solid, powdery, or granular form and the resultant is then incorporated into the food or drink product. Alternatively, the composition of the present disclosure may be mixed or dissolved directly into raw materials of the food or drink product. The composition of the present disclosure may be applied to, coated onto, infiltrated into, or sprayed onto the food or drink product. The composition of the present disclosure may be dispersed uniformly or distributed unevenly in the food or drink product. A capsule or the like containing the composition of the present disclosure may be prepared. The composition of the present disclosure may be wrapped with an edible film or a food coating agent. Alternatively, an adequate excipient or the like may be incorporated into the composition of the present disclosure, and the resultant may be prepared in the form of, for example, a tablet. The food or drink product containing the composition of the present disclosure may further be processed. Such a processed product also falls within the scope of the present invention.

The food or drink product of the present disclosure may be prepared with the use of various types of additives that are commonly used for food or drink products.

As described above, the food or drink product of the present disclosure has useful effects, it is highly safe, and thus there is no concern about side effects. Further, the composition of the present disclosure has a favorable flavor. Even when it is added to a variety of food or drink products, it does not inhibit the flavor of the food or drink product. Accordingly, the resulting food or drink product can be easily subjected to long-term continuous ingestion, and useful effects thereof can be expected for a long period of time.

Further, the composition of the present disclosure can be formulated not only into food or drink products for humans but also into feeds for animals such as livestock (e.g., cattle, pigs, and chickens), racehorses, and pets (e.g., dogs and cats). Feeds are substantially equivalent to food or drink products except that they are given to non-human subjects. Therefore, the descriptions concerning food or drink products above can also be applied to feeds.

Hereafter, the present invention is described in greater detail with reference to the examples, although the present invention is not limited to these examples.

### [Test Example]

### (1) Preparation of lactic acid bacteria

As lactic acid bacteria, *Lactobacillus gasseri* strain CP2305 (Accession Number: FERM BP-11331) and *Lactobacillus gasseri* strain CP2305s (Accession Number: NITE BP-1405) were prepared.

### (2) Measurement of the total serum protein level and the albumin level

A vessel containing blood collected from a subject was mixed by inverting, allowed to stand, and centrifuged to obtain a serum sample. The obtained serum sample was subjected to a colorimetric method (the Biuret method) and another colorimetric method (a modified BCP method) to measure the total serum protein level and the albumin level.

### (3) Measurement of the circumferential length of the triceps muscle of the calf

With the use of an apparatus for measuring the circumferential length, the triceps muscle of the calf of the subject was measured.

### (4) Measurement of levels of the growth hormone and the insulin-like growth factor 1 (IGF-1)

The serum sample obtained from the subject was subjected to a chemiluminescence enzyme immunoassay (CLEIA) method to measure the growth hormone level. The serum sample was subjected to the RIA method to measure the IGF-1 level.

### (5) Measurement of the circumferential length of the gastrocnemius muscle

With the use of an apparatus for measuring the circumferential length, the gastrocnemius muscle of the subject was measured.

### (6) Measurement of the grip strength

With the use of an apparatus for measuring the grip strength, the grip strength was measured.

### [Example 1]

### <Preparation of test beverage>

With the use of *Lactobacillus gasseri* strain CP2305 and *Lactobacillus helveticus* strain, skimmed milk powder and an yeast extract were fermented at 32°C to 37°C for 12 to 22 hours, the resulting fermented milk was supplemented with liquid sugar, a sweetener, an acidulant, a stabilizer, and a flavoring agent, and the resultant was then sterilized.

A placebo beverage was prepared and sterilized in the same manner as described above with the use of milk fermented with *Lactobacillus helveticus* strain under the same conditions while refraining from the use of the strain CP2305.

A paper bottle was filled with 200 ml of the sterilized milk, and the test beverage and the placebo beverage were prepared.

### [Example 2]

### <Test of beverage for human use>

Subjects (34 subjects, middle-aged to older people in their 40s to 90s) were divided into two groups, a bottle of the test beverage containing *Lactobacillus gasseri* strain CP2305 (the amount of bacterial cells: 10¹⁰ cells) or the placebo beverage that does not contain the strain CP2305 was administered every day over the period of 12 weeks.

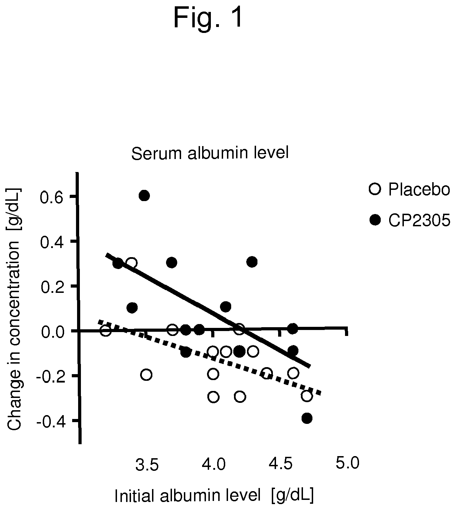

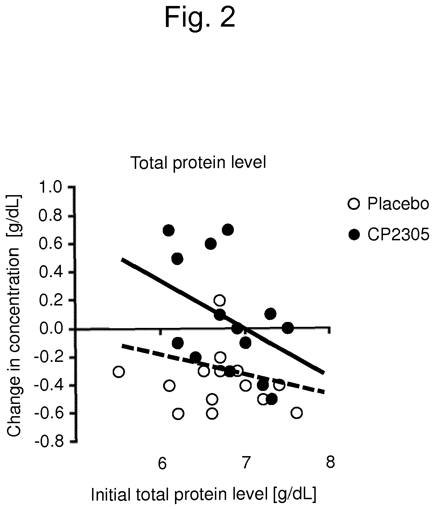

As a result, improvement and change in the total protein level and the albumin level in the serum samples were observed in the group to which the strain CP2305 had been administered (Figs. 1 and 2). Figs. 1 and 2 each show the plot of changes in the serum albumin level and the total serum protein level relative to the initial levels and the results of calculation of the regression lines with the least square method. The results demonstrate that protein metabolism is enhanced in the middle-aged to older subjects.

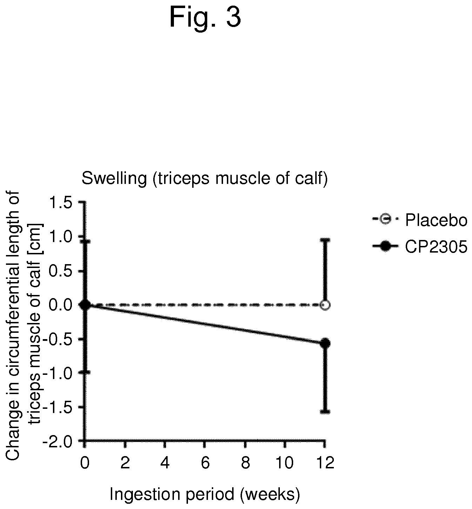

In the subjects in their 70s to 90s of the group to which the strain CP2305 had been administered, decrease was observed in the circumferential length of the triceps muscle of the calf, which is an indicator of swelling, and reduction of swelling was confirmed (Fig. 3). The graph shown in Fig. 3 demonstrates changes in the circumferential length of the triceps muscle of the calf relative to a period of drinking (weeks).

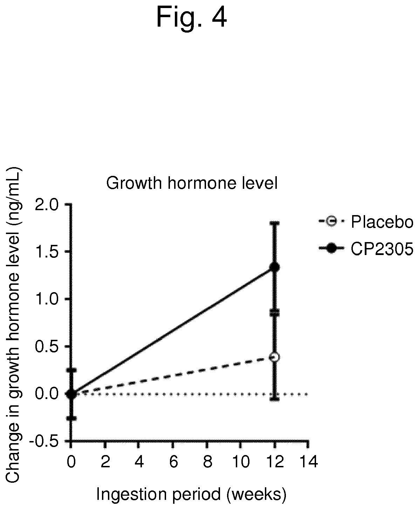

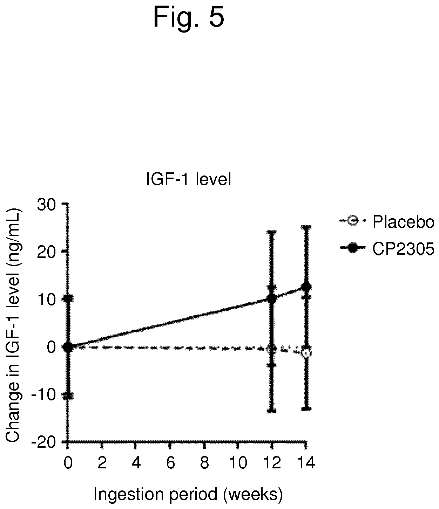

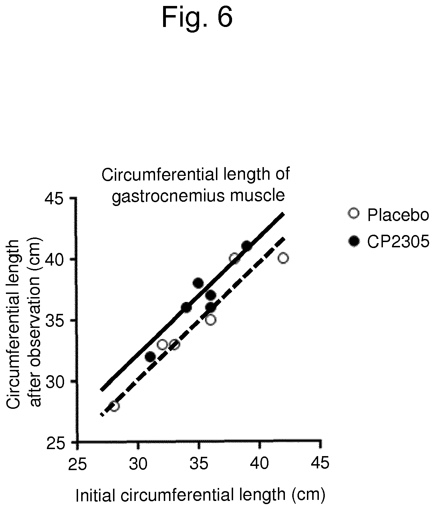

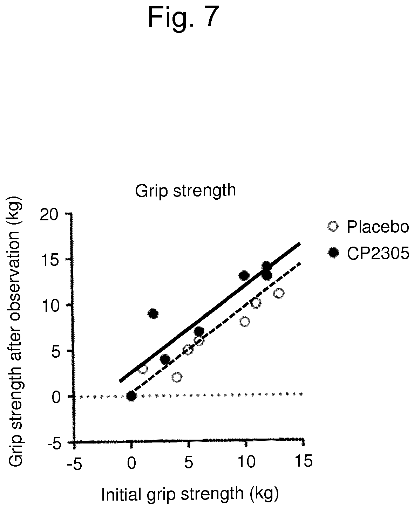

The increased growth hormone level and insulin-like growth factor 1 (IGF-1) level were observed in the group to which the strain CP2305 had been administered (Figs. 4 and 5). The increased circumferential length of the gastrocnemius muscle and the increased grip strength were also observed (Figs. 6 and 7). Figs. 4 and 5 each show changes in the growth hormone level and in the IGF-1 level relative to a period of drinking (weeks). Figs. 6 and 7 each show the plot of changes relative to the initial levels and the results of calculation of the regression lines with the least square method. The results demonstrate that the muscle mass and the muscle strength are increased in the subjects.

### [Accession Numbers]

Accession Number: FERM BP-11331 (*Lactobacillus gasseri* strain CP2305; date of deposition: September 11, 2007)
Accession Number: NITE BP-1405 (*Lactobacillus gasseri* strain CP2305s; date of deposition: August 14, 2012)

## Claims

1. A composition for improving the total serum protein level and/or the serum albumin level comprising, as an active ingredient, lactic acid bacteria and/or a treated product thereof.

2. A composition for improving swelling comprising, as an active ingredient, lactic acid bacteria and/or a treated product thereof.

3. A composition for increasing the muscle mass and/or the muscle strength comprising, as an active ingredient, lactic acid bacteria and/or a treated product thereof.

4. The composition according to any one of claims 1 to 3, wherein the lactic acid bacteria belong to the genus *Lactobacillus.*

5. The composition according to any one of claims 1 to 4, wherein the lactic acid bacteria are *Lactobacillus gasseri.*

6. The composition according to any one of claims 1 to 5, wherein the lactic acid bacteria are *Lactobacillus gasseri* strain CP2305 (Accession Number: FERM BP-11331) or a variant thereof, or *Lactobacillus gasseri* strain CP2305s (Accession Number: NITE BP-1405) or a variant thereof.

7. The composition according to any one of claims 1 to 6, which is used for improving a nutritional status.

8. The composition according to any one of claims 1 to 7, which is administered to human subjects in their 40s or older.

9. The composition according to any one of claims 1 to 8, which is administered to human subjects with the total serum protein level of 6.5 g/dL or lower and/or the serum albumin level of 4 g/dL or lower.

10. The composition according to any one of claims 1 to 9, which is selected from the group consisting of food or drink products, feeds, nutritious supplements, and medicaments.

11. The composition according to claim 10,wherein the food or drink products comprise fermented milk beverages, yogurt, powdered milk, baby foods, *miso* soup, retort foods, and tablets.
